(19) **Europäisches Patentamt / European Patent Office / Office européen des brevets**

(11) **EP 2 110 662 A1**

(12) **EUROPEAN PATENT APPLICATION**
published in accordance with Art. 153(4) EPC

(43) Date of publication:
**21.10.2009 Bulletin 2009/43**

(21) Application number: **08710799.1**

(22) Date of filing: **05.02.2008**

(51) Int Cl.:
*G01N 27/416* (2006.01)      *G01N 27/26* (2006.01)
*G01N 27/327* (2006.01)      *G01N 27/48* (2006.01)
*G01N 37/00* (2006.01)

(86) International application number:
**PCT/JP2008/051867**

(87) International publication number:
**WO 2008/096757 (14.08.2008 Gazette 2008/33)**

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **06.02.2007 JP 2007027222**

(71) Applicant: **Toppan Printing Co., Ltd.
Tokyo 110-8560 (JP)**

(72) Inventor: **NAKAYAMA, Masato
Tokyo 101-0024 (JP)**

(74) Representative: **Williams, Gareth Owen
Marks & Clerk LLP
62-68 Hills Road
Cambridge
CB2 1LA (GB)**

(54) **METHOD FOR DETECTION OF BIOLOGICAL MOLECULE AND CHIP FOR DETECTION OF BIOLOGICAL MOLECULE**

(57)      A biomolecules detection method which detects a detection target biomolecule by a measurement of an electrochemical response, the method including: a standard solution measurement process which measures an electrochemical response of a standard solution which includes an electrochemically reactive agent having a direct or indirect binding capacity to the detection target biomolecule; sample solution measurement process which measures the electrochemical response of a sample solution, the sample solution containing, a sample material containing the detection target biomolecule, and the electrochemically reactive agent; a comparison process which compares an electrochemical response measurement result of the standard solution and an electrochemical response measurement result of the sample solution, and thereby obtains a comparison result; and a biomolecules content information detection process which, based on the comparison result, detects a content information of the detection target biomolecule in the sample solution.

FIG. 1B

**Description**

[Field of the Invention]

The present invention relates to an electrochemical biomolecules detection method in which an electrochemically reactive agent (oxidization-reduction reagent) which can bind to biomolecules, and a biomolecules detection chip used in the aforementioned method.
Priority is claimed on Japanese Patent Application No. 2007-27222, filed February 6, 2007, the content of which is incorporated herein by reference.

[Background Art]

In recent years, in the fields of medical treatments, food hygiene, or environmental preservation, or the like, there has been a demand for technologies by which genetic molecules and proteins can be analyzed easily, and rapidly at a low cost. Conventionally, biomolecules sensors using fluorescent reagents, in particular, photometric detection methods for DNA sensing have been commonly used. However, since expensive fluorescent reagents and fluorescent detection apparatus are used in such technologies, the cost expansion has been unavoidable. Moreover, there have been problems in that pretreatments for fixing capturing molecules for target biomolecules on a solid phase, and operations to regulate the solid phase reaction are necessary, resulting in that the analysis could not be performed rapidly and easily.
On the other hand, there have been proposed methods of electrochemically detecting the biomolecules using electrochemically reactive agents (oxidization-reduction reagents) which can bind to the biomolecules, and also biomolecules detection chip used in such methods. Such electrochemical detection methods do not require expensive reagents, and the reaction operation is relatively easy, acquiring high expectations for applications in the area of point of care (POC).
Japanese Patent Publication No. 3511596 discloses a method in which an electrochemically reactive reagent which specifically binds to double strand DNA is added to the sample solution. The method then measures the electrochemical response therein (change in the solution resistivity), thereby detecting the genetic molecules in the sample solution. More particularly, for the electrochemically reactive reagent (oxidization-reduction reagent), the method adopts Hoechst 33258, a miner groove binder. In the method, a sample including genetic molecules is mixed with the electrochemically reactive reagent and the reagents for the genetic molecules binding reaction. The reaction mixture is subjected to a resistivity comparison before and after the genetic molecules synthesis reaction. Thereby, the genetic molecules can be stably detected even from sample solutions containing a low concentration of the target genetic molecules.
However, in the detection method of aforementioned Japanese Patent Publication No. 3511596, measurements are performed before the genetic molecules synthesis reaction (nucleic acid amplification reaction) and after the genetic molecules synthesis reaction, and then the measurement results are compared (see paragraph 0026 of Japanese Patent Publication No. 3511596). Accordingly, it had been required to prepare two sets of precious samples and expensive reagents, resulting in an increase in the cost. Moreover, in the cases where a constant-temperature synthesis reaction is used, in which the reaction may begin to proceed at the moment the reaction mixture for the genetic molecules synthesis is prepared, as in nucleic acid sequence based amplification (NASBA), isothermal and chimeric primer initiated amplification of nucleic acids (ICAN), loop mediated isothermal amplification (LAMP), the genetic molecules synthesis reaction is not necessarily constant. In such cases, a precise result can not be obtained by comparing the measurement result before and after the genetic molecules synthesis reaction. Moreover, in the genetic molecules detection method described in the Japanese Patent Publication No. 3511596, when an abnormality emerged in the sensor part for detecting the electrochemical response due to the impurities in the reaction mixture, such abnormality can not be detected from the measurement result, resulting in an inability to perform a reliable genetic molecules detection.
The present invention is achieved in view of the aforementioned circumstances, and an object thereof is to provide an electrochemical biomolecules detection method in which a low-cost and reliable detection can be performed, and also to provide a biomolecules detection chip used in such method.

[Means for Solving the Problem]

As a result of intense studies in order to achieve the aforementioned objects, the inventors of the present invention found that by comparing electrochemical responses in a standard solution which contains an electrochemically reactive agent but do not contain biomolecules, and in a sample solution in which the electrochemically reactive agent and the biomolecules are reacted, the biomolecules can be detected in a similar manner with the aforementioned related art. The inventors also found that even when a constant-temperature synthesis reaction (NASBA, ICAN, and LAMP) is adopted, in which the reaction may begin to proceed at the moment the reaction mixture is prepared, a precise comparison can be obtained, thus achieving the present invention.

Namely, the present invention provides a biomolecules detection method which detects a detection target biomolecule by a measurement of an electrochemical response, the method including: a standard solution measurement process which measures an electrochemical response of a standard solution which includes an electrochemically reactive agent having a direct or indirect binding capacity to the detection target biomolecule; a sample solution preparation process which adds a sample material including the detection target biomolecule to the standard solution which has completed the standard solution measurement process, and thereby prepares a sample solution; a sample solution measurement process which measures an electrochemical response of the sample solution; a comparison process which compares an electrochemical response measurement result of the standard solution and an electrochemical response measurement result of the sample solution, and thereby obtains a comparison result; a biomolecules content information detection process which, based on the comparison result, detects a content information of the detection target biomolecule in the sample solution.

The present invention also provides a biomolecules detection method which detects a detection target biomolecule by a measurement of an electrochemical response, the method including: a standard solution measurement process which measures an electrochemical response of a standard solution which includes an electrochemically reactive agent having a direct or indirect binding capacity to the detection target biomolecule; a sample solution preparation process which adds a sample material including the detection target biomolecule to the standard solution which has completed the standard solution measurement process, and thereby prepares a sample solution; a sample solution measurement process which measures an electrochemical response of the sample solution; a comparison process which compares an electrochemical response measurement result of the standard solution and an electrochemical response measurement result of the sample solution, and thereby obtains a comparison result; a biomolecules content information detection process which, based on the comparison result, detects a content information of the detection target biomolecule in the sample solution.

According to the biomolecules detection method of the present invention, since there is no need to prepare two sets of the precious sample and the solution including expensive reagents and the like, compared to the conventional biomolecules detection methods, the cost for the detection is reduced. Moreover, in the cases where a constant-temperature synthesis reaction (NASBA, ICAN, LAMP) is used, in which the reaction may begin to proceed at the moment the reaction mixture is prepared, by using the standard solution, a precise comparison can be performed.

Moreover, according to the biomolecules detection method of the present invention, since the measurement can be performed in a manner not necessarily including an amplification process of the detection target biomolecule, a rapid and low-cost measurement can be performed, resulting in applications in the cases involving broad range of target molecules including molecules not suitable for amplification, or in the cases where amplification is not necessary.

In the biomolecules detection method of the present invention, the sample solution may be composed of two or more than two of the sample solution each of which includes an unknown concentration of the detection target biomolecule; the sample solution measurement process may include electrochemical response measurements of each of the two or more than two of the sample solutions with unknown concentrations; the comparison process may relatively compare the measurement result of the electrochemical response measurement for the standard solution, and each of the measurement results of the electrochemical response measurements for the sample solutions, and thereby obtains a comparison result; and the biomolecules content information extraction process detects the content information of the detection target biomolecule in each of the sample solutions, based on the comparison result.

The biomolecules detection method of the present invention may further include: after the sample solution measurement process, a second standard solution measurement process which measures an electrochemical response of the standard solution or a standard solution having the same composition with the standard solution; and a correction process which, based on a comparison result between the measurement result of the standard solution measurement process and the measurement result of the second standard solution measurement process, corrects the electrochemical response measurement.

According to the aforementioned method, a calibration can be performed by an electrochemical measurement of the standard solution including the electrochemically reactive agent, after performing the biomolecules detection, in the same reaction vessel. Accordingly, an abnormality in the sensor part or the like can be detected, resulting in a measurement with a high reliability.

The biomolecules detection method of the present invention may further include: after the sample solution measurement process, a second standard solution measurement process which measures an electrochemical response of the standard solution or a standard solution having the same composition with the standard solution; and an abnormality detection process which detects an abnormality of a detection member used in the electrochemical response measurement, based on a comparison result of the measurement result of the standard solution measurement with the measurement result of the second standard solution measurement process.

According to the aforementioned method, a calibration can be performed by an electrochemical measurement of the standard solution including the electrochemically reactive agent, after performing the biomolecules detection, in the same reaction vessel. Accordingly, an abnormality in the sensor part or the like can be detected, resulting in a measurement with a high reliability.

In the biomolecules detection method of the present invention, the sample material may be obtained by performing a reaction to a material in which the detection target biomolecule is directly or indirectly amplified and/or denatured.

Even in cases the sample includes a small amount of the detection target, by amplifying it with a PCR reaction or the like, the target can be detected. Moreover, by denaturation processes such as a denaturation of double strand nucleic acid into single strand nucleic acid, the detection can be performed easily.

In the biomolecules detection method of the present invention, the sample solution may include plural kinds of the detection target biomolecule and plural kinds of the electrochemically reactive agent.

In the electrochemical response measurement, electric current value and the voltage value of the solution is measured. In the measurement, due to the characteristic of the electrochemically reactive agent (oxidization-reduction reagent), a peak of the electric current appears within a specific voltage region (electric potential region). Accordingly, for example an electrochemically reactive agent can be introduced, which specifically binds to a plurality of different biomolecules, and shows specific electric potential activities for each of such binding states. Thereby, a specific peak of the electric current appears corresponding to each of the biomolecules, and can be characterized through the electric potential region at the time. Moreover, through the electric current value at the time, a quantitation can be performed. In other words, by performing a single measurement, content information for a plurality of different detection target biomolecules can be detected.

In the biomolecules detection method of the present invention, the electrochemical response measurement in the standard solution measurement process or the sample solution measurement process may be a measurement of a diffusion electric current value.

The electrochemically reactive agent may be a substance selected from the group consisting of: ferrocenyl naphthalene diimide (FND); Hoechst including Hoechst 33258, Hoechst 33342, or the like; distamycin; nuclear yellow; 4', 6-diamidino-2-phenylindole (DAPI); Berenil; methylene blue; mitoxantrone; daunomycin; ethidium bromide; propidium iodide; 9-Aminoacridine; ellipticine; amsacrine; hycanthone; actinomycin D; chromomycin; mithramycin A; echinomycin; quinacrine; nogalamycin; proflavine; amiloride; trioxsalen; and chloroquine.

In the biomolecules detection method of the present invention, the detection target may be a molecule having charge or electronegativity. Moreover, the detection target may be a single or double stranded nucleic acid, a protein, a lipid or a glucide.

In the biomolecules detection method of the present invention, based on a comparison of measurement results of the standard solution and the sample solution, another sample solution containing an unknown concentration of the same biomolecule may be quantitatively evaluated. Those techniques can be applied for a detection of nucleotide substitutions. In the methods such as LAMP, UCAN, Allele Specific Primer (ASP), since base substitutions are identified and thereby nucleic acid amplification is triggered, the amplification amount differs depending on the existence of such substitutions in the genetic molecules. Accordingly, by performing a comparison measurement of a sample solution containing material obtained by amplifying normal genetic molecules and the standard solution, and by performing a measurement of a sample solution containing material obtained by amplifying an unknown genetic molecules, and by evaluating the result in relation with the aforementioned comparison measurement result, it can be determined whether the unknown genetic molecules are normal, or includes substitutions.

In the electrochemical response measurement of the standard solution and the sample solution, the detection can be performed using the same electrode or using individual electrodes, and the measurement can be performed a plural times. The measurement methods may be two-electrode methods which involve two electrodes, i.e., a counter electrode and a working electrode, or three-electrode methods which further involve a reference electrode, or other methods. Among the three-electrode methods, an electricity detection method in which the counter electrode and the working electrode constitute a field transistor.

Moreover, the present invention provides a biomolecules detection chip which detects a detection target biomolecule by an electrochemical response measurement, the chip including: a chamber which is able to accommodate either a standard solution containing an electrochemically reactive agent having a binding capacity to the detection target biomolecule or a sample solution containing a sample material which potentially contains the detection target biomolecule and the electrochemically reactive agent; a liquid introduction device which introduces the standard solution or the sample solution into the chamber; an electrochemical response measurement device which measures an electrochemical response of either the standard solution or the sample solution accommodated in the chamber; and a liquid ejection device which ejects either the standard solution or the sample solution from the chamber.

According to the biomolecules detection chip, the standard solution and the sample solution can be sequentially introduced into the same chamber, and electrochemical responses thereof can be measured and compared. Accordingly, detection target biomolecules can be detected rapidly and in an inexpensive manner.

In the biomolecules detection chip of the present invention, the chamber may further include an amplification/denaturation device which amplifies or denatures the detection target biomolecule in the sample solution.

The amplification/denaturation device may be a temperature control device.

According to the above-mentioned constitution, by amplifying or denaturing the detection target biomolecule, the detection sensitivity can be improved.

[Brief Description of the Drawings]

[FIG. 1A] FIG. 1A is a figure explaining the principle of the biomolecules detection of the present invention.
[FIG. 1B] FIG.1B is a figure explaining the principle of the biomolecules detection of the present invention.
[FIG. 2] FIG. 2 is a figure showing an experiment result of the biomolecules detection method of the present invention, showing the relationship of single stranded oligomer DNA concentration and FND diffusion electric current value.
[FIG. 3A] FIG. 3A is a figure showing an experiment result of the biomolecules detection method of the present invention, showing the relationship of number of human genomic DNA molecules and FND diffusion electric current value.
[FIG. 3B] FIG. 3B is a figure showing an experiment result of the biomolecules detection method of the present invention, showing the relationship of number of human genomic DNA molecules and FND diffusion electric current value.
[FIG. 4A] FIG. 4A is a figure showing an experiment result of the biomolecules detection method of the present invention, showing results of genetic detection by FND diffusion electric current.
[FIG. 4B] FIG. 4B is a figure showing an experiment result of the biomolecules detection method of the present invention, showing results of genetic detection by FND diffusion electric current.
[FIG. 5A] FIG. 5A is a figure showing an experiment result of the biomolecules detection method of the present invention, showing results of genetic detection by FND diffusion electric current.
[FIG. 5B] FIG. 5B is a figure showing an experiment result of the biomolecules detection method of the present invention, showing results of genetic detection by FND diffusion electric current.

[Best embodiments for carrying out the invention]

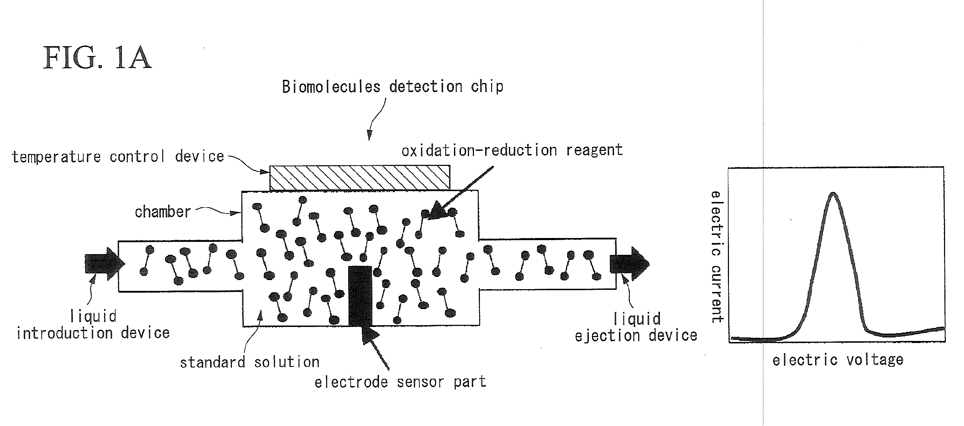

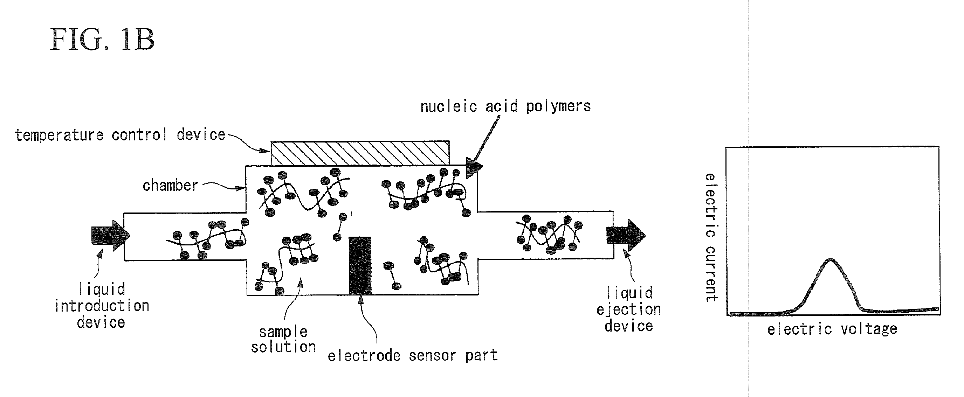

Hereinafter, an embodiment for carrying out the biomolecules detection method and the biomolecules detection chip of the present invention is explained in detail, referring to the attached figures. FIGS 1A to 5B are figures exemplary showing the embodiment of the present invention. In these figures, parts accompanied with the same reference symbols represent the same parts, and the basic constitutions and the functions thereof are the same.
The principles of the biomolecules detection method of the present invention are as follows.
In a reaction vessel as shown in FIG. 1A, which is provided with an electrode sensor part, a conductive solvent containing an oxidization-reduction reagent which can bind to target biomolecules, as a standard solution, is filled. Then an electric current measurement is performed by scanning electric voltage. Since the oxidization-reduction reagent is diffused in the conductive solvent substantially uniformly, the diffusion electric current corresponding to the concentration of the oxidization-reduction reagent is measured.
On the other hand, as shown in FIG. 1B, when target biomolecules exist in the reaction vessel similar to the aforementioned explanation, the oxidization-reduction reagent is trapped by the biomolecules. Accordingly, the oxidization-reduction reagent becomes diffused in an inconsistent manner in the conductive solvent. In this case, when compared to the state shown in FIG. 1A, the amount of the oxidization-reduction reagent exists in the vicinity of the electrode sensor part is decreased. Therefore, in this case, when an electric current measurement is performed by scanning electric voltage, an electric current value lower than the diffusion electric current that corresponds to the oxidization-reduction reagent would be obtained (an electrochemical response measurement).
Accordingly, in the biomolecules detection method of the present invention, first, a conductive solvent including an oxidization-reduction reagent (an electrochemically reactive agent), that is, a standard solution, is filled in the reaction vessel. Then the diffusion electric current value is measured (standard solution measurement process). Next, a conductive solvent containing a detection target material (sample material) and the oxidization-reduction reagent is filled in the reaction vessel. Thereafter, the diffusion electric current value is measured (sample solution measurement process). Next, the difference between the both measurement values is compared (comparison process). Based on this comparison result, the existence of the biomolecules and the contained amount (concentration) thereof can be obtained (biomolecules content information detection process).
In the aforementioned biomolecules detection method, after finishing the standard solution measurement process, a detection target material may be added to the standard solution used in this standard solution measurement process, and thereby a sample solution may be prepared (sample solution preparation process).
In cases wherein the amount of the biomolecules contained in the target material is low, an amplification reaction can be applied. Methods such as LAMP, ICAN, PCR, NASBA may be used for the cases the target biomolecules is nucleic acids. In this case, the diffusion electric current value is measured for the conductive solvent containing the oxidization-reduction reagent, and the conductive solvent containing the oxidization-reduction reagent, an amplification reaction reagent, and the target material. By comparing the both measurement values, biomolecules can be detected.

Furthermore, after these measurements, the diffusion electric current value can be measured again for the conductive solvent containing the oxidization-reduction reagent (second standard solution measurement process). By comparing this result with the previously measured diffusion electric current value for the similar conductive solvent, a calibration of the electrode sensor (correction process) or an abnormality detection may be performed (abnormality detection process).

In the biomolecules detection method of the present invention, when nucleic acids are used as the biomolecules, the electrochemically reactive agent used in the method is not particularly limited, as long as the material directly or indirectly binds to the nucleic acids. For example, the following may be used: ferrocenyl naphthalene diimide (FND); Hoechst including Hoechst 33258, Hoechst 33342, or the like; distamycin; nuclear yellow; 4', 6-diamidino-2-phenylindole (DAPI); Berenil; methylene blue; mitoxantrone; daunomycin; ethidium bromide; propidium iodide; 9-Aminoacridine; ellipticine; amsacrine; hycanthone; actinomycin D; chromomycin; mithramycin A; echinomycin; quinacrine; nogalamycin; proflavine; amiloride; trioxsalen; chloroquine; or the like. Among them, FND or Hoechst 33258 is particularly preferably used.

[Example 1]

As an example of the aforementioned biomolecules detection method of the present invention, the following experiment is performed.

<Electrode preparation>

The electrode surface of a single electrode (gold electrode; 1 mm diameter; BAS incorporation) is ground by diamond grounding (4 to 6 $\mu$m diamond slurry), followed by an elecropollish (cyclic voltammetry) in sulfuric acid solution, using an ALS650 (BAS incorporation). After the grounding, a running water wash using distilled water is performed. On this electrode surface, 2 $\mu$l of mercaptohexanol solution is dropped. The electrode is then placed in a humidity maintained box, and incubated for two hours. The electrode is washed with running distilled water immediately prior to the use, and subjected to the experiment.

<FND solution preparation>

Acetic acid is added to ferrocenyl naphthalene diimide and stirred to dissolve. To this solution, potassium acetate, potassium chloride is added and sufficiently stirred to dissolve, and prepared to obtain an appropriate concentration with distilled water.

<DPV measurement>

Measurement environment: measurement device / ALS650 (BAS incorporation)
Three-electrode method measurement: the working electrode was a single electrode; the counter electrode was platinum wire; the reference electrode was silver/silver chloride Measurement condition: Differential Pulse Voltammetry (DPV) Initial voltage = 0 mV, final voltage = 600 mV, voltage increase = 20 mV, amplitude = 50 mV, pulse length = 0.05 sec Sampling duration = 0.0167 sec, pulse duration = 0.2 sec, stationary duration = 2 sec, sensitivity = le-6A/V.

<Experiment 1>

Using two single electrodes after masking treatment (electrode preparation), the FND diffusion electric current of the following three preparation solutions (300 $\mu$L) was measured by DPV.

    1. FND solution 270 $\mu$L + DDW 30 $\mu$L
    2. FND solution 270 $\mu$L + 10 pmol/$\mu$L single stranded oligomer DNA(120 mer) 30 $\mu$L
    3. FND solution 270 $\mu$L + 100 pmol/$\mu$L single stranded oligomer DNA(120 mer) 30 $\mu$L
DDW is double distilled water. For the single stranded oligomer DNA, a product of Operon Biotechnology Company was used.

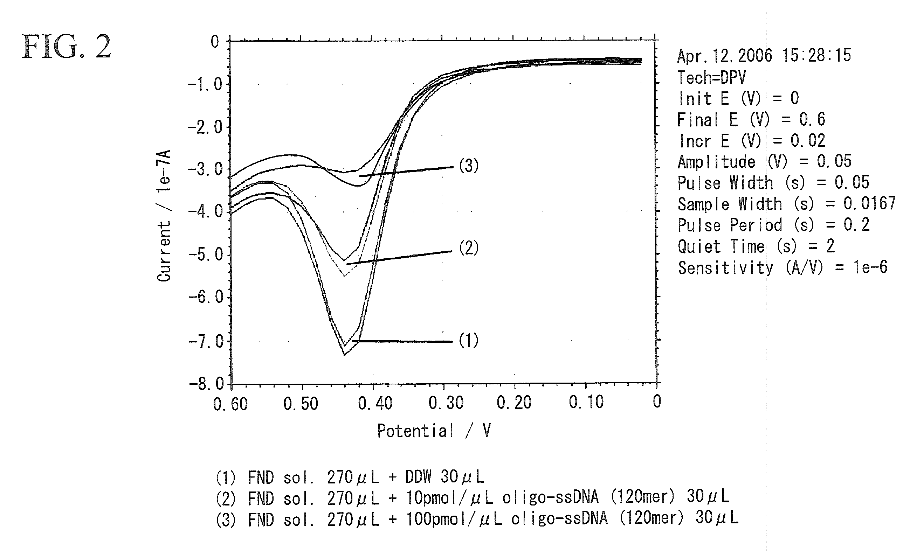

The results of this experiment are shown in FIG. 2. From this graph, it is confirmed that as the concentration of the single stranded oligomer DNA in the preparation solution increases, there is a tendency that the diffusion electric current value decreases.

<Experiment 2>

Using two single electrodes after masking treatment, the FND diffusion electric current of the following eight preparation solutions (300 μL) was measured by DPV.

1. FND solution 150 μL + human genomic DNA (0 copy) solution 150 μL
2. FND solution 150 μL + human genomic DNA (10 copies) solution 150 μL
3. FND solution 150 μL + human genomic DNA ($10^2$ copies) solution 150 μL
4. FND solution 150 μL + human genomic DNA ($10^3$ copies) solution 150 μL
5. FND solution 150 μL + human genomic DNA ($10^4$ copies) solution 150 μL
6. FND solution 150 μL + human genomic DNA ($10^5$ copies) solution 150 μL
7. FND solution 150 μL + human genomic DNA ($10^6$ copies) solution 150 μL
8. FND solution 150 μL + human genomic DNA ($10^7$ copies) solution 150 μL

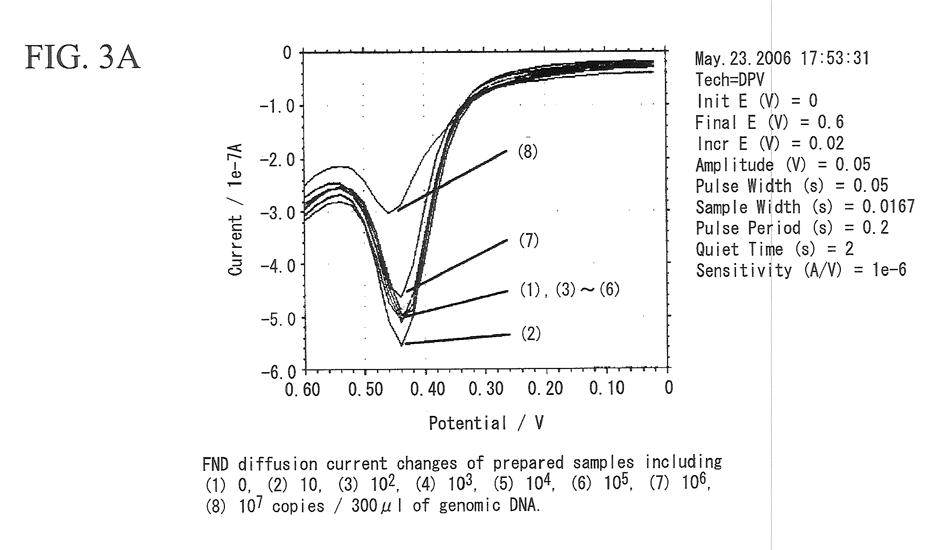

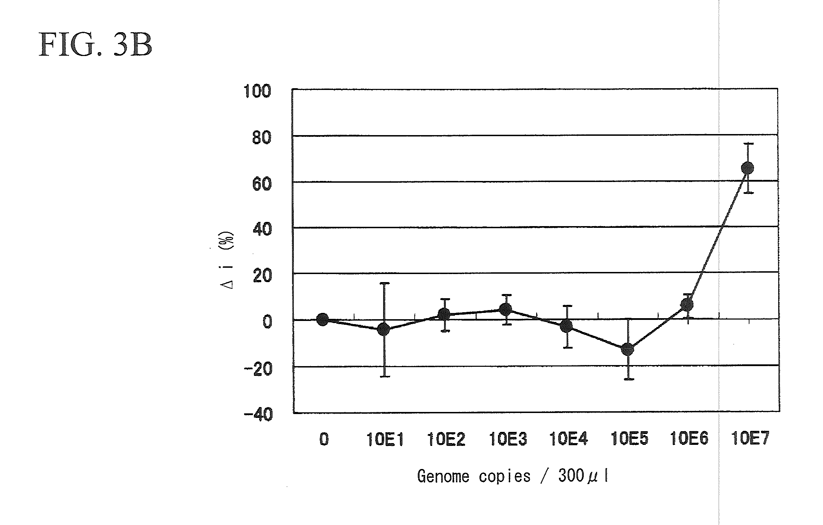

For the human genomic DNA solution, Human Genome DNA from Roche was used.
The results of this experiment are shown in FIGS. 3A and 3B.
FIG. 3B shows diffusion electric current value change rate Δi (%) of the preparation solution shown by the following equation. In the equation; the diffusion electric current value of the preparation solution in which the number of genomic DNA molecule is zero copy, is represented by diffusion electric current value i0; and the diffusion electric current value under existence of various number of genomic molecules is represented by i. The following equation calculates diffusion electric current value variation ratio Δi (%) of the respective preparation solution.

$$\Delta i(\%) = [\ (i0 - i)\ /\ i0\ ] \times 100$$

From those graphs, it can be understood that the diffusion electric current value measured for the preparation solutions containing the human genomic DNA at the concentrations of $10 \sim 10^5$ copies/300μL (2 to 6 of the aforementioned list) is approximately the same as the measurement value for the preparation solution containing no human genomic DNA. On the other hand, it was confirmed that for the preparation solutions containing the human genomic DNA at the concentrations of higher than $10^6$ copies/300μL, depending on the concentration, the diffusion electric current value decreases. As a result, the detection limit for the detection of human genomic DNA by the biomolecules detection method of the present invention was clarified.

<Experiment 3>

Using two single electrodes after masking treatment, the FND diffusion electric current of the following three preparation solutions (300 μL) was measured by DPV.

1. FND solution 270 μL + DDW 30μL
2. FND solution 270 μL + unreacted PCR mixture 30μl
3. FND solution 270 μL + reacted PCR mixture 30μl
The unreacted PCR mixture is the solution prepared as shown bellow, containing the human genomic DNA as the amplification target, and the PCR reagents. The reacted PCR mixture is the solution obtained by applying PCR reaction to the unreacted PCR mixture, using the condition shown bellow.

[Table 1]

| < PCR sol. > | | | < PCR reaction > | | |
|---|---|---|---|---|---|
| Beta3-F (20mer:100pmol/μL) | 1.5 μL | (final 5pmol/μL) | 95°C | 3 min | |
| Beta3-R (19mer:100pmol/μL) | 1.5 μL | (final 5pmol/μL) | 95°C | 30 sec. | |
| 8mM dNTP Mix (2mM each) | 3.0 μL | | 65°C | 30 sec. | 50 cycles |
| 10 X PCR buffer | 3.0 μL | | 72°C | 30 sec. | |
| human genome (200ng/μL) | 1.0 μL | (final $6.7 \times 10^4$ copies) | | | |
| Taq polymerase (unit) | 1.0 μL | | | | |
| DDW | 19.0 μL | | *PCR product = 200 bp | | |

to 30.0 μL

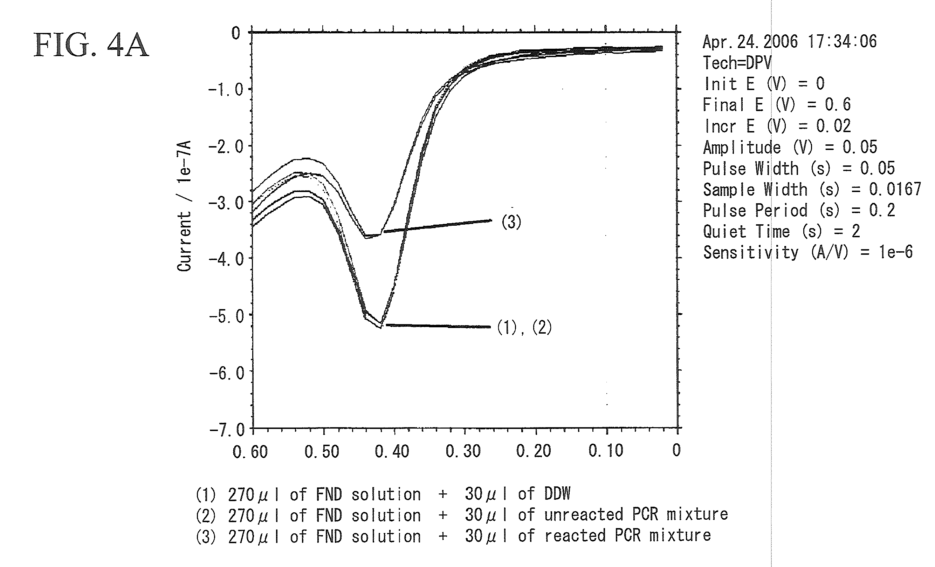

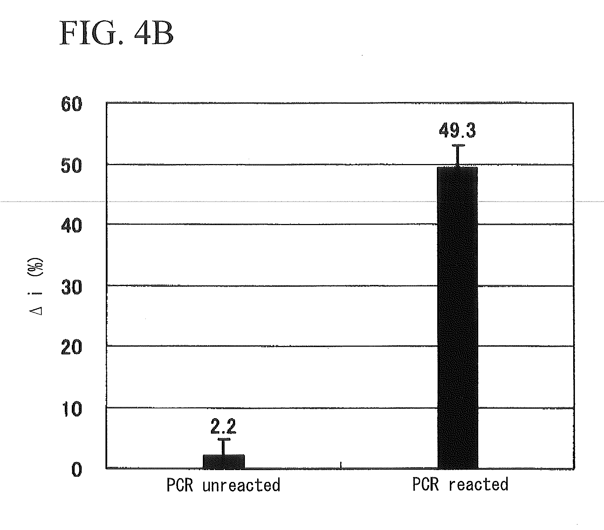

The results of this experiment are shown in FIGS. 4A and 4B. FIG. 4B shows the diffusion electric current value variation ratio $\Delta i$ (%) calculated by the same method as for FIG. 3B. From these graphs, it can be understood that the diffusion electric current value of the preparation solution in which the unreacted PCR mixture is added to the FND solution (2 of the aforementioned list) is approximately the same as the diffusion electric current value of the preparation solution in which distilled water is added to the FND solution (1 of the aforementioned list). On the other hand, compared to those cases, the diffusion electric current value of the preparation solution in which the reacted PCR mixture is added to the FND solution (3 of the aforementioned list) was notably lower. As a result, it was found that, by comparing the diffusion electric current value of the preparation solution which contains only the FND solution, and the diffusion electric current value of the preparation solution in which the reacted PCR mixture is added to the FND solution, the target biomolecules in the preparation solution can be detected.

<Experiment 4>

Using one single electrode after masking treatment, the FND diffusion electric current of the following three preparation solutions (300 μL) was measured by DPV, at the freezing temperature (4 °C).

    1. FND solution 200 μL + DDW 100μL
    2. FND solution 200 μL + 200ng/μL human genomic DNA solution 100μL
    3. FND solution 200 μL + 200ng/μL denatured human genomic DNA solution 100μL

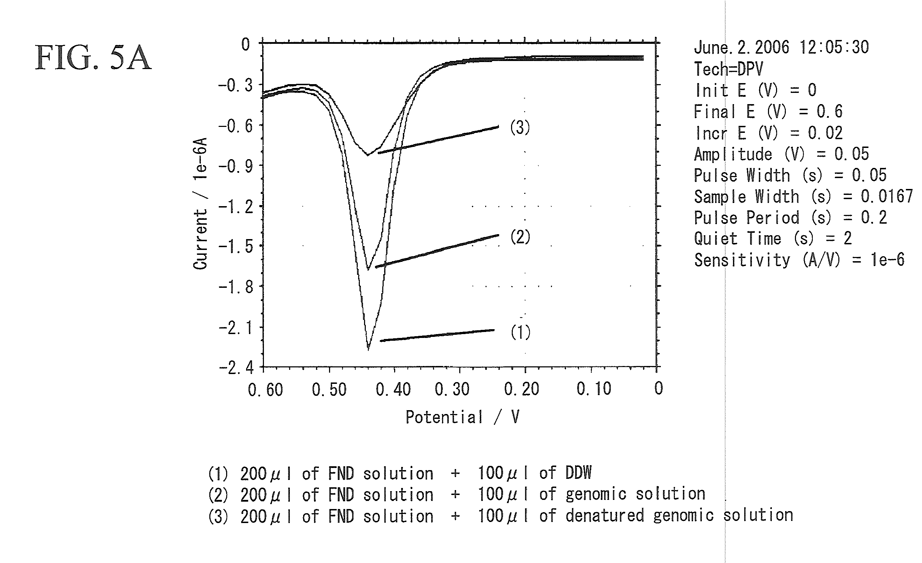

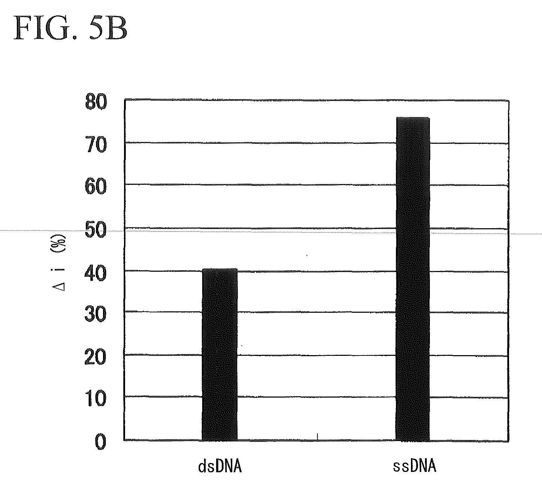

For the above-listed preparation solutions 1 to 3, the DPV measurement was performed after leaving each of the preparation solution under 4 °C for 10 minutes. For preparation solution 2, double strand human genomic DNA is used. On the other hand, for preparation solution 3, 100μL of the solution containing the same double strand human genomic DNA is heated at 95 °C for 5 minutes, followed by a rapid cooling treatment in which the sample is incubated in ice water for 3 minutes. Thereafter, preparation solution 3 is obtained by mixing the resulting solution with 200μL of the FND solution. By these treatments, the human genomic DNA in preparation solution 3 had been denatured into single strands. The results of this experiment are shown in FIGS. 5A and 5B. FIG. 5B shows the diffusion electric current value variation ratio $\Delta i$ (%) calculated by the same method as for FIG. 3B. From these graphs, it was found that, as compared to the preparation solution in which distilled water is added to the FND solution (1 of the aforementioned list), the preparation solution containing human genomic DNA solution or the denatured human genomic DNA solution (2, 3 of the aforementioned list) showed lower diffusion electric current value. It was also found that, as compared to the preparation solution containing double strand human genomic DNA (2 of the aforementioned list), the preparation solution in which the double strand human genomic DNA was denatured into single strands (3 of the aforementioned list) showed higher variation ratio of the diffusion electric current value, relative to the preparation solution containing only the FND solution.

[Example 2]

On the other hand, as an example of the biomolecules detection chip of the present invention, a biomolecules detection chip can be used, which includes: a chamber which is able to accommodate either a standard solution containing an electrochemically reactive agent having a binding capacity to the detection target biomolecule or a sample solution containing a sample material which potentially contains the detection target biomolecule and the electrochemically reactive agent; a liquid introduction device which introduces the standard solution or the sample solution into the chamber; an electrochemical response measurement device which measures an electrochemical response of either the standard solution or the sample solution accommodated in the chamber; and a liquid ejection device which ejects either the standard solution or the sample solution from the chamber.

The chamber may further include a denaturation device which amplifies or denatures the detection target biomolecule in the sample solution. The denaturation device may be a temperature control device.

By using aforementioned chip, the amplification of the detection target biomolecule by the temperature control device and the electrochemical measurement can be performed in the single chamber. Accordingly, the experiment procedure can be simplified, and the amount of used reagents can be reduced to the minimum level.

Although the biomolecules detection method and the biomolecules detection chip of the present invention are explained above by showing specific embodiments, the present invention is not limited to those. A person skilled in the art may add various modifications or improvements in the detection methods or the components of the chip, within the scope of the essence of the present invention.

[Industrial Applicability]

The biomolecules detection method and the biomolecules detection chip of the present invention can be applied as technologies to analyze the biomolecules such as genetic molecules or proteins at a low cost as well as easily and rapidly, in the industrial fields such as medical treatments, food hygiene, or environmental reservation.

**Claims**

1. A biomolecules detection method which detects a detection target biomolecule by a measurement of an electrochemical response, the method comprising:

   a standard solution measurement process which measures an electrochemical response of a standard solution which includes an electrochemically reactive agent having a direct or indirect binding capacity to the detection target biomolecule;
   sample solution measurement process which measures the electrochemical response of a sample solution, the sample solution containing, a sample material containing the detection target biomolecule, and the electrochemically reactive agent;
   a comparison process which compares an electrochemical response measurement result of the standard solution and an electrochemical response measurement result of the sample solution, and thereby obtains a comparison result; and
   a biomolecules content information detection process which, based on the comparison result, detects a content information of the detection target biomolecule in the sample solution.

2. A biomolecules detection method which detects a detection target biomolecule by a measurement of an electrochemical response, the method including:

   a standard solution measurement process which measures an electrochemical response of a standard solution which includes an electrochemically reactive agent having a direct or indirect binding capacity to the detection target biomolecule;
   a sample solution preparation process which adds a sample material including the detection target biomolecule to the standard solution which has completed the standard solution measurement process, and thereby prepares a sample solution;
   a sample solution measurement process which measures an electrochemical response of the sample solution;
   a comparison process which compares an electrochemical response measurement result of the standard solution and an electrochemical response measurement result of the sample solution, and thereby obtains a comparison result;
   a biomolecules content information detection process which, based on the comparison result, detects a content information of the detection target biomolecule in the sample solution.

3. The biomolecules detection method according to claims 1 or 2, wherein

the sample solution is composed of two or more than two of the sample solution each of which includes an unknown concentration of the detection target biomolecule;

the sample solution measurement process includes electrochemical response measurements of each of the two or more than two of the sample solutions with unknown concentrations;

the comparison process relatively compares the measurement result of the electrochemical response measurement for the standard solution, and each of the measurement results of the electrochemical response measurements for the sample solutions, and thereby obtains a comparison result; and

the biomolecules content information extraction process detects the content information of the detection target biomolecule in each of the sample solutions, based on the comparison result.

**4.** The biomolecules detection method according to claims 1 or 2, further comprising:

a second standard solution measurement process, after the sample solution measurement process, measures an electrochemical response of the standard solution or a standard solution having the same composition with the standard solution; and

a correction process which, based on a comparison result between the measurement result of the standard solution measurement process and the measurement result of the second standard solution measurement process, corrects the electrochemical response measurement.

**5.** The biomolecules detection method according to claims 1 or 2, further comprising:

a second standard solution measurement process which, after the sample solution measurement process, measures an electrochemical response of the standard solution or a standard solution having the same composition with the standard solution; and

an abnormality detection process which detects an abnormality of a detection member used in the electrochemical response measurement, based on a comparison result of the measurement result of the standard solution measurement with the measurement result of the second standard solution measurement process.

**6.** The biomolecules detection method according to claims 1 or 2, wherein
the sample material is obtained by performing a reaction to a material in which the detection target biomolecule is directly or indirectly amplified and/or denatured.

**7.** The biomolecules detection method according to claims 1 or 2, wherein
the sample solution includes plural kinds of the detection target biomolecule and plural kinds of the electrochemically reactive agent.

**8.** The biomolecules detection method according to claims 1 or 2, wherein
the electrochemical response measurement in the standard solution measurement process or the sample solution measurement process is a measurement of a diffusion electric current value.

**9.** The biomolecules detection method according to claims 1 or 2, wherein
the electrochemically reactive agent is a substance selected from the group consisting of: ferrocenyl naphthalene diimide (FND); Hoechst including Hoechst 33258, Hoechst 33342, or the like; distamycin; nuclear yellow; 4', 6-diamidino-2-phenylindole (DAPI); Berenil; methylene blue; mitoxantrone; daunomycin; ethidium bromide; propidium iodide; 9-Aminoacridine; ellipticine; amsacrine; hycanthone; actinomycin D; chromomycin; mithramycin A; echinomycin; quinacrine; nogalamycin; proflavine; amiloride; trioxsalen; and chloroquine.

**10.** The biomolecules detection method according to claims 1 or 2, wherein
the detection target is a molecule having charge or electronegativity.

**11.** The biomolecules detection method according to claims 1 or 2, wherein
the detection target is nucleic acid, protein, lipid or glucide.

**12.** The biomolecules detection method according to claim 10, wherein
the detection target is nucleic acid.

**13.** The biomolecules detection method according to claim 12, wherein
the detection target is single strand nucleic acid.

**14.** The biomolecules detection method according to claim 12, wherein
the detection target is double strand nucleic acid.

**15.** A biomolecules detection chip which detects a detection target biomolecule by an electrochemical response measurement, the chip comprising:

a chamber which is able to accommodate either a standard solution containing an electrochemically reactive agent having a binding capacity to the detection target biomolecule, or a sample solution containing a sample material and the electrochemically reactive agent;
a liquid introduction device which introduces the standard solution or the sample solution into the chamber;
an electrochemical response measurement device which measures an electrochemical response of either the standard solution or the sample solution accommodated in the chamber;
and a liquid ejection device which ejects either the standard solution or the sample solution from the chamber.

**16.** The biomolecules detection chip according to claim 15, wherein
the chamber further comprises a temperature control device which amplifies or denatures the detection target biomolecule in the sample solution.

# FIG. 1A

Biomolecules detection chip

temperature control device

oxidation-reduction reagent

chamber

standard solution

electrode sensor part

liquid
introduction device

liquid
ejection device

electric current

electric voltage

FIG. 1B

FIG. 2

Apr. 12. 2006 15:28:15
Tech=DPV
Init E (V) = 0
Final E (V) = 0.6
Incr E (V) = 0.02
Amplitude (V) = 0.05
Pulse Width (s) = 0.05
Sample Width (s) = 0.0167
Pulse Period (s) = 0.2
Quiet Time (s) = 2
Sensitivity (A/V) = 1e-6

(1) FND sol. 270 $\mu$L + DDW 30 $\mu$L
(2) FND sol. 270 $\mu$L + 10pmol/$\mu$L oligo-ssDNA (120mer) 30 $\mu$L
(3) FND sol. 270 $\mu$L + 100pmol/$\mu$L oligo-ssDNA (120mer) 30 $\mu$L

FIG. 3A

May. 23. 2006 17:53:31
Tech=DPV
Init E (V) = 0
Final E (V) = 0.6
Incr E (V) = 0.02
Amplitude (V) = 0.05
Pulse Width (s) = 0.05
Sample Width (s) = 0.0167
Pulse Period (s) = 0.2
Quiet Time (s) = 2
Sensitivity (A/V) = 1e-6

FND diffusion current changes of prepared samples including
(1) 0, (2) 10, (3) $10^2$, (4) $10^3$, (5) $10^4$, (6) $10^5$, (7) $10^6$,
(8) $10^7$ copies / 300 $\mu$ l of genomic DNA.

FIG. 3B

Genome copies / 300 μl

EP 2 110 662 A1

# FIG. 4A

Apr. 24. 2006 17:34:06
Tech=DPV
Init E (V) = 0
Final E (V) = 0.6
Incr E (V) = 0.02
Amplitude (V) = 0.05
Pulse Width (s) = 0.05
Sample Width (s) = 0.0167
Pulse Period (s) = 0.2
Quiet Time (s) = 2
Sensitivity (A/V) = 1e-6

(1) 270 μl of FND solution + 30 μl of DDW
(2) 270 μl of FND solution + 30 μl of unreacted PCR mixture
(3) 270 μl of FND solution + 30 μl of reacted PCR mixture

EP 2 110 662 A1

FIG. 4B

FIG. 5A

June. 2. 2006 12:05:30
Tech=DPV
Init E (V) = 0
Final E (V) = 0.6
Incr E (V) = 0.02
Amplitude (V) = 0.05
Pulse Width (s) = 0.05
Sample Width (s) = 0.0167
Pulse Period (s) = 0.2
Quiet Time (s) = 2
Sensitivity (A/V) = 1e-6

(1) 200 $\mu$ l of FND solution + 100 $\mu$ l of DDW
(2) 200 $\mu$ l of FND solution + 100 $\mu$ l of genomic solution
(3) 200 $\mu$ l of FND solution + 100 $\mu$ l of denatured genomic solution

EP 2 110 662 A1

FIG. 5B

## INTERNATIONAL SEARCH REPORT

| | International application No. |
|---|---|
| | PCT/JP2008/051867 |

A. CLASSIFICATION OF SUBJECT MATTER
*G01N27/416*(2006.01)i, *G01N27/26*(2006.01)i, *G01N27/327*(2006.01)i,
*G01N27/48*(2006.01)i, *G01N37/00*(2006.01)i

According to International Patent Classification (IPC) or to both national classification and IPC

B. FIELDS SEARCHED

Minimum documentation searched (classification system followed by classification symbols)
G01N27/416, G01N27/26, G01N27/327, G01N27/48, G01N37/00

Documentation searched other than minimum documentation to the extent that such documents are included in the fields searched
| Jitsuyo Shinan Koho | 1922-1996 | Jitsuyo Shinan Toroku Koho | 1996-2008 |
| Kokai Jitsuyo Shinan Koho | 1971-2008 | Toroku Jitsuyo Shinan Koho | 1994-2008 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>Y | Masaaki KOBAYASHI et al., "DNA stick -POC Taiogata DNA Sensor no Kaihatsu-", Chemical Sensors, 12 September, 2002 (12.09.02), Vol.18 SupplementB(2002), pages 55 to 57 | 1-3,6,8-14<br>4-5,7,15-16 |
| Y | JP 3511596 B2 (Eichi TAMIYA), 16 January, 2004 (16.01.04), Full text; all drawings & JP 2002-218998 A | 15-16 |
| Y | JP 2006-504936 A (Radiometer Medical A/S), 09 February, 2006 (09.02.06), Full text; all drawings & WO 2004/040284 A1 & US 2004/0132193 A1 & EP 1558921 A1 | 4-5 |

☒ Further documents are listed in the continuation of Box C. ☐ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 27 February, 2008 (27.02.08) | 11 March, 2008 (11.03.08) |

| Name and mailing address of the ISA/ | Authorized officer |
|---|---|
| Japanese Patent Office | |
| Facsimile No. | Telephone No. |

Form PCT/ISA/210 (second sheet) (April 2007)

**INTERNATIONAL SEARCH REPORT**

International application No.

PCT/JP2008/051867

C (Continuation).   DOCUMENTS CONSIDERED TO BE RELEVANT

| Category* | Citation of document, with indication, where appropriate, of the relevant passages | Relevant to claim No. |
|---|---|---|
| Y | JP 2005-518810 A  (Molecular Sensing PLC.), 30 June, 2005 (30.06.05), Par. No. [0023] & US 2005/0221315 A1     & GB 205455 D & EP 1481083 A2         & EP 1808494 A1 & WO 2003/074731 A2     & DE 60310532 D & AT 348896 T           & DK 1481083 T | 7 |

Form PCT/ISA/210 (continuation of second sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2007027222 A

- JP 3511596 B